# EUROPEAN PATENT SPECIFICATION

(11) **EP 3 454 833 B1**
(45) Date of publication and mention of the grant of the patent: **10.05.2023**
(21) Application number: 17796990.4
(22) Date of filing: 12.05.2017
(51) Int. Cl.: A61K 9/00, A61K 31/135, A61K 45/00, A61K 45/06, A61P 35/00, A61P 43/00

(54) **METHODS FOR TREATING LIVER TISSUE**
VERFAHREN ZUR BEHANDLUNG VON LEBERGEWEBE
PROCÉDÉS DE TRAITEMENT DU TISSU HÉPATIQUE

(30) Priority: 13.05.2016 US 201662336498 P
(43) Date of publication of application: 20.03.2019
(73) Proprietor: Teclison Limited, Grand Cayman KY1-1208 (KY)
(72) Inventor: LEE, Ruey-min, Short Hills, NJ 07078 (US)
(74) Representative: Mewburn Ellis LLP
(86) International application number: PCT/US2017/032524
(87) International publication number: WO 2017/197342

(56) References cited:
- US-A1- 2014 065 139
- US-A1- 2014 371 709
- US-A1- 2015 258 125
- AKINAGA SONODA ET AL: "Enhanced Antitumor Effect of Tirapazamine Delivered Intraperitoneally to VX2 Liver Tumor-Bearing Rabbits Subjected to Transarterial Hepatic Embolization", CARDIOVASCULAR AND INTERVENTIONAL RADIOLOGY., vol. 34, no. 6, 9 April 2011 (2011-04-09), pages 1272-1277, XP055595895, US ISSN: 0174-1551, DOI: 10.1007/s00270-011-0156-4

## Description

### CROSS-REFERENCE

This PCT application claims priority to U.S. Provisional Patent Application No. 62/336,498, filed on May 13, 2016.

### BACKGROUND OF THE INVENTION

Liver cancer can be derived from the liver or from metastatic cancers of other organs, such as from colorectal, breast or lung cancers. The most common type of liver cancer is hepatocellular carcinoma (HCC), which accounts for about 75 percent of all liver cancers. Other types of liver cancer include fibrolamellar hepatocellular carcinoma, cholangiocarcinoma, and angiosarcoma.

Liver cancer is the fifth leading cause of cancer-related death in the world. The highest incidence rates in the world are in Asia, which accounts for the majority of all known incidences of liver cancer worldwide. In Asia, particularly high incidence rates can be seen in China, Korea, and Japan, where some registries show incidences rates of greater than 20/100,000 as of 2011. In the United States, the incidence of liver cancer has almost tripled since the early 1980s, where it is the fastest rising cause of cancer-related deaths. The prognosis for liver cancer is very poor because of its rapid growth and absence of symptoms during the early stages.

Various available methods for treating liver cancer suffer from a number of profound drawbacks. Non-targeted therapies for liver treatment, such as conventional chemotherapy and radiation therapy, are not particularly effective and present risks including systemic exposure of chemotherapeutic agents and ionizing radiation. Targeted therapies still yield a vast diversity of side effects including: rashes, hand-foot syndrome, diarrhea, fatigue, high blood pressure, hair loss, nausea, itching, low white blood cell count, poor appetite, vomiting, bleeding, increased amylase/lipase blood counts, low phosphorus level, constipation, shortness of breath.

### SUMMARY OF THE INVENTION

There exists a considerable need for alternative compositions and methods for treating a variety of liver cancers. The present invention addresses this need and provides other related advantages as well. The invention is defined by the claims.

The invention provides a hypoxia-activated bioreductive agent for use in a method of administering said agent to a liver tissue of a subject in need thereof to treat liver cancer, the method comprising: applying locally to the liver tissue a therapeutically effective amount of a hypoxia-activated bioreductive agent, wherein applying is performed when pressure of a hepatic artery of the subject is reduced, wherein said pressure is reduced by introducing a vessel occlusion device comprising a balloon into said hepatic artery, thereby treating said liver cancer. In some embodiments, the pressure is reduced proximal to the liver tissue. In some embodiments, the local application is performed in the hepatic artery. In some embodiments, the pressure of the hepatic artery is reduced by at least about 20%. In some embodiments, the pressure of the hepatic artery is reduced by reducing hepatic artery blood flow volume by at least about 20%.

In some embodiments, the pressure of the hepatic artery is reduced by introducing a vessel occlusion device into the hepatic artery. In some embodiments, the pressure of the hepatic artery is reduced by introducing a balloon into the hepatic artery. In some embodiments, the pressure of the hepatic artery is reduced by inflating the balloon.

In some embodiments, the local infusion is performed with the aid of a catheter. In some embodiments, the catheter comprises an external diameter of 0.5 mm or less. In some embodiments, the catheter is a balloon catheter. Non-limiting examples of balloons and balloon catheters are described below. In some embodiments, the hypoxia-activated bioreductive agent is (i) applied from a distal tip of the catheter, and (ii) diffused to the liver tissue.

In some embodiments, the pressure of the hepatic artery of the subject is reduced prior to applying the hypoxia-activated bioreductive agent. In some embodiments, the pressure of the hepatic artery of the subject is reduced concurrent with applying the hypoxia-activated bioreductive agent.

Some embodiments further comprise embolizing the liver tissue. In some embodiments, the step of embolizing takes place after the step of applying of the hypoxia-activated bioreductive agent. In some embodiments, the liver tissue is embolized with an absorbable or non-absorbable embolization agent selected from the group consisting of ethiodized oil, absorbable gelatin/Gelfoam, and non-absorbable microspherical beads.

In some embodiments, the hypoxia-activated bioreductive agent is tirapazamine. In some embodiments, about 0.5 mg to about 500 mg of tirapazamine are applied to the subject.

In some embodiments, the liver tissue comprises cancer.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1 is a schematic illustration of a balloon catheter.
FIG. 2 is a schematic illustration of local infusion of a hypoxia-activated bioreductive agent to liver tissue when pressure is reduced.

### DETAILED DESCRIPTION OF THE INVENTION

Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods, provided that the invention is covered by the claims. Unless stated otherwise, the present invention is not limited by the illustrated ordering of acts or events, as some acts may occur in different orders and/or concurrently with other acts or events. Furthermore, not all illustrated acts or events are required to implement a methodology in accordance with the present invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

"Treatment", "treating", "palliating" and "ameliorating", as used herein, are used interchangeably. These terms refer to an approach for obtaining beneficial or desired results including but not limited to therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder.

The term "effective amount" or "therapeutically effective amount" refers to that amount of a hypoxia-activated bioreductive agent described herein that is sufficient to effect the intended application including but not limited to disease treatment. The therapeutically effective amount may vary depending upon the intended application (in vitro or in vivo), or the subject and disease condition being treated, e.g., the weight and age of the subject, the severity of the disease condition, the manner of administration and the like, which can readily be determined by one of ordinary skill in the art. The term also applies to a dose that will induce a particular response in target cells, e.g., hypoxia and cell death. The specific dose will vary depending on the particular compounds chosen, the dosing regimen to be followed, whether it is administered in combination with other compounds, timing of administration, the tissue to which it is administered, and the physical delivery system in which it is carried.

A "region of hypoxia", "hypoxic environment", or "hypoxic condition" as used herein, are used interchangeably. These terms refer to the level of oxygen within the region is at least lower or less than about 10%, and preferably lower or less than about 5%. For example, the level of oxygen in the hypoxic region may be about 10, 9, 8, 7, 6, 5, 4, 3, 2, or 1%. Generally, an oxygen level of about 10% or lower, and preferably about 5% or lower, is sufficient to activate hypoxia-activated bioreductive agents such as tirapazamine to a level that is at least 10-fold more active than the prodrug form. Those of skill in the art are familiar with the measurement of oxygen levels in biological systems, and are also aware that oxygen measurements may be expressed in "mm Hg", wherein, for example, 10% O₂ is equal to 76 mmHg and 1% O₂ is equal to 7.6 mmHg.

In one aspect, the disclosure provides a hypoxia-activated bioreductive agent for use in a method of administering said hypoxia-activated bioreductive agent to a liver tissue of a subject in need thereof as further specified in claim 1. The method comprises applying locally to the liver tissue a therapeutically effective amount of a hypoxia-activated bioreductive agent, wherein applying is performed when pressure of a hepatic artery of the subject is reduced. In some embodiments pressure is reduced proximal to the liver tissue. In some embodiments, local application is performed in the hepatic artery. In further embodiments, the blood pressure and/or blood flow volume in the hepatic artery are reduced. In some embodiments, pressure is reduced by introducing a vessel occlusion device into the hepatic artery. In general, arterial blood pressure may be reduced prior to or concurrent with local infusion of a hypoxia-activated bioreductive agent into the hepatic artery. In some embodiments, blood flow slows to increase the duration of tissue exposure to the locally applied hypoxia-activated bioreductive agent.

In any of the various aspects, the hypoxia-activated bioreductive agent is applied locally to the liver tissue. Local application may be performed by applying the hypoxia-activated bioreductive agent to the liver tissue without systemic infusion. A wide variety of approaches can be used to locally apply the hypoxia-activated bioreductive agent. Non-limiting examples of local application include: topical application to liver tissue, injection into the intraperitoneal cavity, direct needle injection of liver tissue, direct infusion into an artery, direct infusion into an artery that supplies the liver, direct infusion into the hepatic portal vein, and direct infusion into a hepatic artery. In some instances, local application may refer to infusion into an arterial branch that supplies liver tissue. Local application may refer to infusion into a hepatic arterial branch that supplies a target region in the liver tissue, such as a cancer lesion. For the purposes of this application, an application may be local when an infusion of a hypoxia-activated bioreductive agent into the hepatic artery is sufficiently close to a targeted region of liver tissue such that the applied hypoxia-activated bioreductive agent diffuses to the targeted region of the liver tissue. Where the agent is infused directly into a hepatic artery branch, application may be sufficiently close to the liver tissue such that the hypoxia-activated bioreductive agent diffuses to a targeted region of the liver tissue. In some instances, the hypoxia-activated bioreductive agent may be locally applied by direct infusion into a hepatic artery branch proximal to liver tissue.

A wide variety of devices may be used for local application. Non-limiting examples of such devices include: a catheter, a balloon catheter, a microcatheter, a guidewire, a balloon, a needle, and a syringe. Application devices may inject the hypoxia-activated bioreductive agent into the liver tissue directly. Application devices may inject the hypoxia-activated bioreductive agent into the intraperitoneal cavity where it then diffuses to the liver. In some embodiments, devices may directly infuse the hypoxia-activated bioreductive agent into an artery where it then diffuses to liver tissue. A device, such as for example a catheter, may be used to cannulate arterial branches that supply the liver tissue. A device may cannulate hepatic artery branch that supplies the liver tissue to apply the hypoxia-activated bioreductive agent. A device may cannulate a hepatic arterial branch supplying a specific liver tissue region or lesion and apply a hypoxia-activated bioreductive agent. A device may cannulate the hepatic artery and apply the hypoxia activated bioreductive agent proximal to the liver tissue and distal from a vessel occlusion device.

Various techniques may be used to visualize local application to liver tissue. Non-limiting examples of visualization technologies include: arteriogram, magnetic resonance imaging, X-ray, cone beam computed tomography, positron emission tomography, and computerized axial tomography. As a specific non-limiting example, an arteriogram may be performed to ensure that a catheter or other device is appropriately placed in the arterial branch that supplies the targeted liver tissue region.

In all aspects, hepatic artery pressure is reduced. In various embodiments, hepatic arterial pressure may be reduced by at least about 5%, 10%, 15%, 20%, 25%, 30%, 35%, 40%, 45%, 50%, 55%, 60%, 65%, 70%, 75%, 80%, 85%, 90%, 95%, or 100%. Local infusion may occur during or after arterial pressure is reduced by about 5% - 100%. In some embodiments, local infusion may occur during or after arterial pressure is reduced by at least about 20%.

In embodiments, pressure of the hepatic artery is reduced by reducing hepatic artery blood flow volume. Blood flow volume may be reduced by at least about 5%, 10%, 15%, 20%, 25%, 30%, 35%, 40%, 45%, 50%, 55%, 60%, 65%, 70%, 75%, 80%, 85%, 90%, 95%, or 100%. Local infusion may occur during or after blood flow volume is reduced by about 5% - 100%. In some embodiments, local infusion may occur during or after blood flow volume is reduced by at least about 20%.

In some embodiments, intra-arterial blood pressure and flow may be monitored using various systems and methods. To monitor intra-arterial blood pressure, an intravascular pressure monitoring system may be used. Intravascular monitoring systems may include pressure wires that sense and measure intravascular pressure using an electronic pressure transducer. Intravascular pressure monitoring systems can comprise an optical coherence tomography (OCT) system with integrated pressure measurement. In some embodiments a pressure transducer may be an optical pressure transducer. A pressure transducer may comprise a semiconductor pressure sensor. A pressure transducer may be a ferro-fluid type pressure transducer. A flow transducer may also be used to measure intravascular flow rate. Pressure and flow measurements may be taken from a single instrument with flow transducer portions and pressure transducer portions. Pressure and flow sensors may be delivered to the site of occlusion.

In various aspects, the hypoxia-activated bioreductive agent is applied into the hepatic artery when pressure of the hepatic artery reduced. In some embodiments, hepatic arterial pressure may be reduced locally using a vessel occlusion device that slows blood flow and reduces blood pressure.

In some embodiments, vessel occlusion devices may be introduced into a hepatic artery to reduce arterial blood pressure and/or flow volume distal from the vessel occlusion device. A wide variety of vessel occlusion devices may be used to decrease arterial pressure. Vessel occlusion devices do not increase or promote arterial flow. Vessel occlusion devices may comprise various instruments or techniques to stop or slow blood flow. Non-limiting examples of vessel occlusion devices are: catheters, balloons, filters, coils, clips, slings, guidewires, intraluminal stents, vascular stents, sutures, embolic filters, cannulae, plugs, drug delivery devices, tissue patch devices, patches, arterial lines, portosystemic shunts and shunts for ascites, occlusion devices to selectively deliver a means to obstruct or fill a passage or space, centering mechanisms for transluminal instruments like catheters, and absorbable or non-absorbable embolization agents.

The vessel occlusion device may be an expandable member. A wide variety of expandable members will be recognized as suitable to sufficiently occlude the hepatic artery to reduce blood pressure and/or flow. Non-limiting examples of vessel occlusion devices having expandable members may include, but are not limited to, the following: a balloon, an expandable catheter, a stent, a stent-graft, a self-expanding construct, a balloon expandable construct, a combination self-expanding and balloon expandable construct, a graft or a mechanical, radially expanding device which may be expanded, for example, via application of a torsional or longitudinal force. Expandable members can also include those which expand due to pneumatic or hydraulic pressure, those which expand due to magnetic forces, those which expand due to the application of energy (for example thermal, electrical, or ultrasonic (piezoelectric) energy). Expandable members can be placed temporarily in any lumen (e.g. a hepatic artery) by expanding said device and then removed by collapsing said device by a torsional or longitudinal force. According to the invention, the vessel occlusion device comprises a balloon.

Various vessel occlusion devices, including an expandable member such as a balloon, may be used to reduce arterial pressure. In various embodiments, pressure of a hepatic artery is reduced by introducing a balloon into the hepatic artery. A balloon may be used alone or combined with another vessel occlusion device. Non-limiting examples of other vessel occlusion devices with which a balloon may be combined include: a wide variety of catheters, filters, coils, guidewires, and intraluminal stents. A balloon may be introduced into a hepatic artery vessel alone or as a component of a balloon catheter instrument.

In some embodiments the pressure of the hepatic artery is reduced by inflating the balloon. In some embodiments, the balloon is inflated for a period of time. A period of time can be from about five seconds or less to up to ten minutes, ten seconds or less to up to ten minutes, fifteen seconds or less to up to ten minutes, twenty seconds or less to up to ten minutes, thirty seconds or less to up to ten minutes.

Balloons may comprise various polymeric materials. Non-limiting examples of polymeric materials that may be used in a vessel occlusion device include: styrenic olefinic rubber and hydrogenated isoprene, polyurethane material, silicone-polyurethane copolymer, diene polymer, a polyamide/polyether block copolymer, polyamides, and any other materials recognized as suitable. by one skilled in the art.

In one embodiment, balloon may be formed from a polyurethane material. An exemplary polyurethane material may comprise a thermoplastic, aromatic, polyether polyurethane synthesized from methylene disocyanate (MDI), polytetramethylene ether glycol (PTMEG) and 1,4 butanediol chain extender. A suitable polyurethane material may have a Shore durometer of at least about 65D, an elongation at break of at least about 300%, and a high tensile strength at yield of about 10,000 psi. Other grades of polyurethane materials may be used, including for example those having a Shore hardness of about D75. Other suitable compliant polymeric materials may include an ethylene alpha-olefin polymer. Other polyurethane materials such as thermoplastic polymers, elastomeric silicones, and latexes may be used.

A balloon may be configured to withstand high interior or exterior pressures. Such a balloon may optionally be reinforced with fibers or webs of fibers. The balloon material may be crosslinked or uncrosslinked. In some embodiments, the polyurethane balloon materials are not crosslinked. The final inflated balloon size can be controlled by crosslinking the balloon material. Conventional crosslinking techniques can be used including thermal treatment and E-beam exposure.

In one embodiment, balloon is formed from a low tensile set polymer such as a silicone-polyurethane copolymer. The silicone-polyurethane may be an ether urethane and more specifically an aliphatic ether urethane. Non-limiting exemplary embodiments may include silicone polyether urethane copolymers, and aliphatic ether urethane cosiloxanes.

In some balloon embodiments, the low tensile set polymer may be a diene polymer. A variety of suitable diene polymers can be used such as but not limited to an isoprene such as an AB and ABA poly(styrene-block-isoprene), a neoprene, an AB and ABA poly(styrene-block-butadiene) such as styrene butadiene styrene (SBS) and styrene butadiene rubber (SBR), and 1,4-polybutadiene. The diene polymer can be an isoprene including isoprene copolymers and isoprene block copolymers such as poly(styrene-block-isoprene). Various neoprene grades may be used as well.

In another balloon embodiment, the polymeric material is a compliant material such as, but not limited to, a polyamide/polyether block copolymer (commonly referred to as PEBA or polyether-block-amide). The polyamide and polyether segments of the block copolymers may be linked through amide or ester linkages. The polyamide block may be selected from various aliphatic or aromatic polyamides known in the art. Some non-limiting examples include nylon 12, nylon 11, nylon 9, nylon 6, nylon 6/12, nylon 6/11, nylon 6/9, and nylon 6/6. The polyether block may be selected from various polyethers known in the art. Some non-limiting examples of polyether segments include poly(tetramethylene glycol), tetramethylene ether, polyethylene glycol, polypropylene glycol, poly(pentamethylene ether) and poly(hexamethylene ether). Various techniques for forming a balloon from polyamide/polyether block copolymer are described in the art, such as in U.S. Pat. No. 6,406,457 to Wang.

In another embodiment, the balloon material is formed from polyamides. The polyamide may have substantial tensile strength, be resistant to pin-holing even after folding and unfolding, and be generally scratch resistant, such as those disclosed in U.S. Pat. No. 6,500,148 to Pinchuk. Some non-limiting examples of polyamide materials suitable for the balloon include nylon 12, nylon 11, nylon 9, nylon 69 and nylon 66.

The balloon can be composed of a single polymeric layer, or alternatively, can be a multilayered balloon, such as those described in U.S. Pat. No. 5,478,320 to Ishida, U.S. Pat. No. 5,879,369 to Trotta, or U.S. Pat. No. 6,620,127 to Lee. The balloon may be composite wherein the balloon has multiple layers. Some balloons may have an overlayer balloon or an underlayer balloon. Each of the layers may be composed of a different polyamide or polyamide/polyether block copolymer.

The balloon may be configured take on various conformations and withstand varying levels of pressure. The balloon may be sufficiently compliant to mold to the anatomy of the blood vessel.

Balloon shape and dimension may be configured to achieve a desired degree of occlusion thus blood pressure reduction. In some instances, the balloon shape can be designed to avoid contact with the blood vessel wall during inflation. In this manner, the blood can flow between the surface of the balloon and the blood vessel wall such that the rate of blood inflow into the liver tissue would be controlled. The effect of shape includes (1) minimizing contact during occlusion, and (2) modulating the flow rate in a manner to allow flow (seeping or creeping) but not perfusion. A balloon may have greater degrees of curvature, i.e., low surface to volume ratio, such as but not limited to a spherical balloon. The shape of balloon can be spherical, cylindrical, or polygonal. Various polymers may be selected for the formation of balloon, as are described above and identified by one skilled in the art. The balloon surface can include gel coatings, surfactant coatings, or be coated to achieve desired design parameters such as vessel contact and increased force against the arterial wall. In this respect, a gel coated balloon surface may be chosen to design to provide greater contact against the vessel wall during delivery of the beneficial agent.

The balloon is configured for hepatic artery vasculature. The balloon may have a length of greater than about 2 mm, 4 mm, 6 mm, 8 mm, 10 mm. In some embodiments the length of the balloon may be from 2 mm to 30 mm. The balloon may have a diameter of at least about 1 mm or greater.

In some embodiments, the balloon may be sized to occlude the hepatic artery to reduce pressure in the hepatic artery. In some embodiments, the reduction in pressure is proximal to liver tissue. In some embodiments, the balloon may be one-size fits all balloon designed for use within a variety of different vessels, such as coronary, peripheral, spinal, cerebral, so as to provide a device for which the operator need not select a pressure or volume for inflation of the balloon. In this manner, the balloon may occlude a blood vessel having a diameter, from about 1 mm to about 2 mm, about 2 mm to about 5 mm, about 2 mm to about 30 mm depending on the desired degree of occlusion. As a non-limiting example, a balloon may elongate when it is inflated within a narrow sized vessel, and may have a spherical shape when it inflated within a larger or wider blood vessel. The balloon may be capable of molding to the blood vessel.

In some embodiments, pressure of a hepatic artery is reduced by inflating a balloon. The balloon may be inflated using various inflation fluids. Inflation fluid may include any pressurized fluid. Examples of inflation fluids include but are not limited to such as carbon dioxide, noble gases including helium, neon, and pressurized liquids such as saline or contrast agents.

A balloon may be inflated to various pressures. Balloon inflation pressures may be about at least 1 atmosphere, 2 atmospheres, 3 atmospheres, 4 atmospheres, 5 atmospheres, or 6 atmospheres. Balloon inflation pressure may be about 1 atmosphere to about 6 atmospheres. Balloon inflation pressure may be about 10 to 16 atmospheres. Balloon inflation pressure may be about 1 atmosphere to about 16 atmospheres.

In various embodiments, local application to the liver tissue may be performed with the aid of a catheter. A catheter may also be used to occlude the hepatic artery thereby reducing blood pressure. A wide variety of catheters may be used to locally apply a hypoxia-activated bioreductive agent, reduce arterial blood pressure, or both. Non-limiting examples of catheters include, but are not limited to, central venous catheters, peripheral intravenous catheters, haemodialysis catheters, as coated catheters, implantable venous catheters, tunnelled venous catheters, catheters useful for angiography, angioplasty, or ultrasound procedures in peripheral veins and arteries, hepatic artery infusion catheters, peripherally inserted central venous catheters (PIC lines), balloon-tipped catheters, total parenteral nutrition catheters, chronic dwelling catheters, peritoneal dialysis catheters, CPB catheters (cardiopulmonary bypass), and microcatheters.

In some embodiments, the catheter can be an intravascular catheter. A wide variety of intravascular catheters may be used in the hepatic artery. Intravascular catheters may be configured for various interventional procedures. Intravascular catheters may be configured for interventional radiology procedures such as trans-arterial embolization. One skilled in the art will recognize that intravascular catheters may have a wide variety of features and/or constructions. Non-limiting examples of intravascular catheters include: balloon catheters, atherectomy catheters, drug delivery catheters, stent delivery catheters, diagnostic catheters and guide catheters, angiocatheters, balloon angiocatheters, coated catheters, microcatheters, balloon microcatheters, closed end catheters, catheters having a plurality of needles, balloon catheters having a plurality of needles, catheters having an outboard projected needle to deliver a therapeutic agent, and soaker catheters.

A person of ordinary skill in the art will be familiar with different types of catheters appropriate for practicing multiple embodiments of the invention(s).

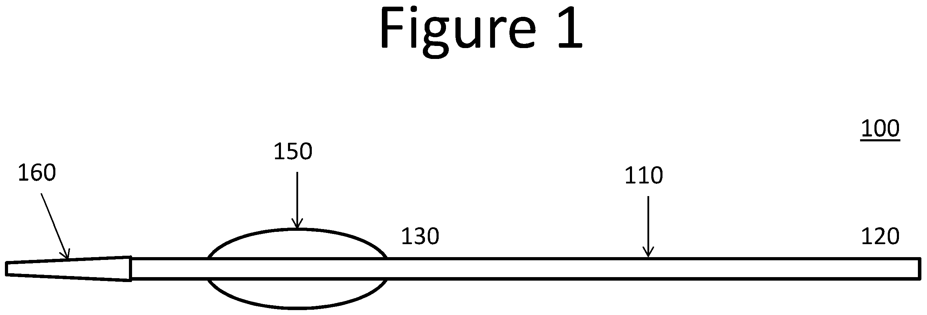

In accordance with one embodiment illustrated in FIG. 1, the catheter 100 includes a generally elongate tubular shaft 110 having a proximal shaft segment 120 and a distal shaft segment 130 in fluid communication. The proximal shaft segment 120 and distal shaft segment 130 can be formed from material having the same or similar hardness or durometer to provide a uniform flexibility along the catheter body. Alternatively, the proximal shaft segment and distal shaft segment can be formed from materials having different flexibilities to provide a catheter having a varied flexibility along a length thereof. As a non-limiting example, the proximal shaft segment may be formed from a hypotube and the distal shaft can be formed from a polymeric material to provide increased flexibility along the catheter tubular shaft. As such, the proximal shaft and distal shaft segments can be formed from the same tube or alternatively can be multiple separate tubes connected or welded together to form a unitary tube. The catheter may comprise one or more polymers or polymer blends having different stiffness.

An elongate shaft 110 may include an inflation lumen configured to provide a passage or flow of inflation fluid to or from an expandable member, such as for example a balloon 150 disposed at or near the distal end 130 of the catheter shaft. The elongate shaft 110 can be formed in a number of shapes, such as for example a tubular or elliptical configuration. However, as would be recognized in the art, other shapes can be employed.

The elongate shaft 110 can further include guidewire lumen, for example, in addition to the inflation and deflation lumen. In this regard, guidewire lumen can be configured to extend from a tip 160 at the distal end of elongate shaft 110 to a more proximal location of the elongate shaft 110 to provide an over-the-wire catheter.

In accordance with another embodiment, elongate shaft 110 can further include a drug delivery lumen, such as for example, a drug infusion lumen configured to locally deliver beneficial agents such as the hypoxia-activated bioreductive agents described above or other agents. In one embodiment, the hypoxia-activated bioreductive agent is locally applied proximally to liver tissue. The hypoxia-activated bioreductive agent may be applied proximally to the liver tissue through the drug delivery lumen from the distal tip of the catheter from which it may diffuse to liver tissue. The catheter may lack a drug delivery lumen and instead, a drug coated balloon may be disposed on the catheter shaft for local delivery of a hypoxia-activated bioreductive agent. In yet other embodiments, the catheter itself may occlude the vessel and the hypoxia-activated bioreductive agent may be locally applied to the liver issue from the distal tip of the catheter.

The elongate shaft 110 may include multiple separate and independent lumens. For example in a balloon catheter, an elongate shaft may include four separate and independent lumens including: an inflation lumen, a deflation lumen, a guidewire lumen, and a drug delivery lumen. However, other configurations can be employed. The diameters of the lumen can have different sizes. For example, in some embodiments, a deflation lumen may have a diameter of about twice the size of an inflation lumen diameter.

The elongate shaft of a catheter 110 can further include a distal tip 160 having a proximal end abutting or overlapping the distal end 130 of the catheter body. The catheter tip 160 can include one or more lumen.

Elongate shaft 110 can be produced from a variety of materials, including metal, plastic and composite materials. In one embodiment, proximal shaft 120 is manufactured as a metal tube, for example, as a stainless steel hypotube, and may be coated with a polymeric material such as PTFE. The metal tube may also be covered with a single or multilayered plastic material through one or more processes, including coextrusion, dipping, heat-shrinking, and electrostatic and thermal coating. In another embodiment, elongate shaft 110 is manufactured as a plastic tube. Materials suitable for use in the catheter tube include, but are not limited to, Polyurethanes (PU), such as Tecoflex, Pellethene, Bionate, corethane, Elasteon, and blends thereof; Polyethylenes (PE), such as PET, PBT, PVDF, Teflon, ETFE, and blends thereof, Polyolefins, such as HDPE, PE, LDPE, LLDPE, Polypropylene, and blends thereof, Polyimides; Polyamides; all classes of Nylons, such as Nylon 11, Nylon 12, Nylon 6,6, Nylon 6, Nylon 7,11, Nylon 11,12, and blends thereof); block copolymers; PEBA-types polymers, such as ELY, PEBAX, Ubesta, and blends thereof, and biodegradable polymers.

Suitable materials also include blends of the above mentioned materials as well as any composite materials, like dual-layers, tri-layers and multi-layers thereof. For example, catheter shaft may be produced from a tube comprising an outer layer made of Nylon and an inner layer made of a lubricious material such as polyethylene or PTFE. A metallic or nonmetallic braiding may also be included within or between layers of the catheter shaft.

In some embodiments, a hypoxia-activated bioreductive agent is applied locally from a distal tip of a catheter and diffused into liver tissue. A catheter tip 160 can be configured to provide atraumatic contact between elongate shaft 110 and a wall against which elongate shaft 110 may be pushed during a surgical procedure. The catheter tip can be configured as a soft tip, which in some embodiments, can be composed of a soft sleeve that is affixed on and that extends beyond distal end 130, or, alternatively, that is affixed on and extends beyond the lumen of elongate shaft 110.

In some embodiments, local application in the hepatic artery is performed with a balloon catheter. The balloon 150 may be inflated to occlude the artery thereby reducing arterial pressure. Arterial pressure may be reduced proximal to the liver tissue. In general, balloon catheter may have an elongate shaft, an expandable member, and a fluid circuit. The fluid circuit may be closed. A balloon catheter may have an elastomeric balloon near the distal tip for the partial or total occlusion of a vessel. Possible conformations and materials of various balloons are described above and are herein incorporated by reference.

Suitable catheters may come in a range of sizes. In some embodiments, the catheter size is 3 French, or about 1 millimeter in external diameter, or smaller. In some embodiments, the catheter size is 4 French or smaller. A combination of catheters of various sizes may be used to occlude an artery and administer a hypoxia-activated bioreductive agent to liver tissue. As a non-limiting example, a 4-French catheter may be used to cannulate a major branch of a hepatic artery that supplies a targeted liver cancer lesion and then a smaller catheter, such as a 3 French or smaller microcatheter, may then be inserted though the 4-French catheter already in place to further cannulate the hepatic artery.

In some instances a smaller catheter, such as for example a microcatheter may allow more distal cannulation. For greater distal cannulation, a microcatheter may further cannulate the arterial branch that supplies a targeted cancer lesion. More distal cannulation may allow for more selective targeting of a tumor lesion. In some embodiments where liver tissue is embolized, greater distal cannulation increases the likelihood that complete blockage of a target arterial branch will be achieved. For instance, in some embodiments where a balloon catheter is used to effectuate a decrease in blood pressure distal from a vessel occlusion device and proximal to the liver tissue, the balloon catheter may be a microballoon catheter. In some embodiments the microballoon catheter may have dimensions of about 3-French or smaller.

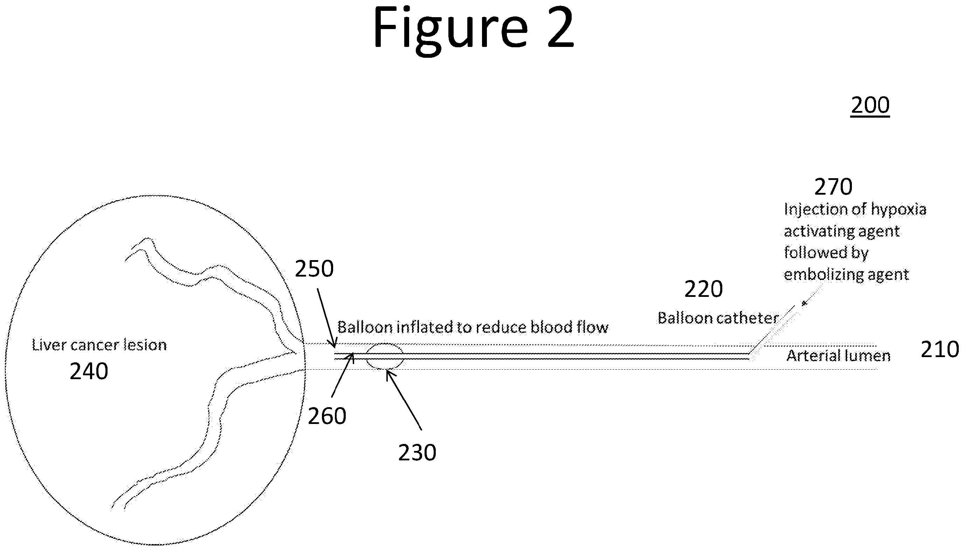

In FIG. 2 an exemplary embodiment 200 illustrates local application into a hepatic artery 210 to a liver tissue lesion 240 with a balloon catheter 220. After inserting a balloon microcatheter and ensuring that the tip 250 of the catheter is positioned appropriately for injection of a hypoxia-activated bioreductive agent, the balloon may be inflated to stop or slow blood flow to reduce the pressure of the artery at and/or beyond the balloon and/or at the distal lumen 260 and distal tip 250 of the catheter. The hypoxia-activated bioreductive agent is then injected 270 into the catheter lumen such that it is delivered to a region of reduced pressure in the hepatic artery distal from the catheter tip 250 and proximal to liver tissue. The applied hypoxia-activated bioreductive agent diffuses to liver tissue. In this non-limiting exemplary embodiment, the liver tissue may be embolized following local infusion. Embolization may be accomplished by injecting an embolization agent through the catheter lumen to the region of reduced blood pressure. The region of reduced blood pressure in the hepatic artery may be proximal to the liver tissue. Embolization agent injection may be monitored under real time x-ray monitoring to ensure that blood flow is blocked and tumor hypoxia induced. The balloon 230 may then deflated after embolization agent injection.

Although discussed with specific reference to catheters, the embodiments of the invention can be applicable to almost any medical device having a vessel occlusion device.

In some embodiments, catheter placement may be visualized with numerous imaging techniques. Catheter placement may be performed using fluoroscopic or non- fluoroscopic x-ray guidance. Cone beam computed tomography may be used to visualize a vessel occlusion device or any radiopaque reagents.

For fluoroscopic placement, X-ray opaque material may be placed on a catheter shaft. For balloon catheters, radiopaque material may be used to indicate the proximal end and distal end of the working length of the balloon. Additionally, radiopaque material may be blended into the polymer matrix of the catheter components.

Vessel occlusion devices may also be visualized using magnetic resonance imaging (MRI). A balloon catheter may be configured for visualization with MRI. MRI visible materials may include ferromagnetic materials such as iron oxide.

Contrast agents or dyes may be used to facilitate visualization. For magnetic resonance imaging, contrast agent may be gadolinium-based. Gadolinium (Gd(III))-based contrast agents may comprise small molecular Gd(III) chelates with high stability. Small molecular Gd(III) chelates have a relatively low relaxivity and extravasate nonselectively from blood into the interstitium of both normal tissue and tumor. Non-limiting examples of molecular Gd(III) chelates include: gadopentetate dimeglumine, Gd-DTPA; gadoterate, Gd-DOTA; gadoteridol, Gd(HP-DO3A); gadodiamide, Gd(DTPA-BMA); Gd-BOPTA; Gd(EOB-DTPA); MS-325. Small molecular Gd(III) contrast agents may be targeted against specific tissues or tissue lesions, such as tumors. Gd(III) based contrast agents may also include macromolecular Gd(III) complexes. Macromolecular Gd(III) complexes may improve relaxivity and pharmacokinetics of Gd(III) based agents. Gd(III) chelates may be attached to macromolecules. For example, Gd(III) ion may be conjugated to polylysine. Macromolecular MRI contrast agents may be prepared by conjugating Gd-DOTA and Gd-DTPA to biocompatible macromolecules. Various bifunctional chelates of DTPA and DOTA may comprise functional groups such as anhydride, N-hydroxysuccinimide activated carboxylic acid, maleimide, alkynyl and azide for conjugation. Additionally, macromolecules may comprise small molecular Gd chelates bifunctional ligands attached to many biocompatible macromolecules such as proteins, polymers and dendrimers. Gd(III)-based contrast agents may also be protein based. For example, Gd(III) may be attached to albumin (e.g., Albumin-(Gd-DTPA)ₓ). Macromolecular Gd(III) complexes may be linear polymer-based. Examples of linear polymer that may be used include but are not limited to: polylysine, polyethylene glycol, and dextran. Gd(III) contrast agents may be dendrimer based. Dendrimers are highly branched macromolecules with precise three-dimensional nanosized molecular structures, which provide a platform for numerous biomedical applications. Dendrimers have many advantages, including precise molecular structure and a large number of surface functionalities for bioconjugation, as compared to linear polymers. Non-limiting examples of possible dendrimeric Gd(III) complexes include Gd-DTPA conjugated to poly(amidoamine) (PAMAM), polypropylene imine) (PPI), or polylysine (PLL). Dendrimer based contrast agents may also be targeted to a specific tissue or tissue lesion, such as cancer. Gd(III) biomolecular conjugates may be biodegradable. Biodegradable Gd(III) complexes may comprise one or more disulfide bonds. Gd(III) chelates may be loaded to liposomal particles via covalently attachment to their surface or encapsulation into their interior core. The size, surface charge, as well as the mechanical properties of nanoparticles can be modulated to optimize their biodistribution and pharmacokinetics and to promote their tumor accumulation for effective cancer MRI. Liposomal contrast agents may be targeted to specific tissues or tissue lesions such as cancer.

Contrast agents or dyes may be used to facilitate visualization using X-ray computed tomography (CT scans) or X-ray fluoroscopy. CT contrast agents may comprise a wide variety of compositions. CT contrast agents may be lathanide-based. CT contrast agents may be iodine based. Iodine-based CT contrast agents may be small molecules and may be ionic or non-ionic. Some small-molecule iodinated contrast agents may be low molecular weight (< 2000 Da) iodinated aromatics. Multiple iodinated aromatic rings may be covalently joined together via a linker. Non-limiting examples of small-molecule iodinated contrast agents include: iohexol; iopromide; iodixanol; ioxaglate; iothalamate; iosimenol; iopamidol; GE-145; 1,3,5-trialkyl-2,4,6-triodobenzenes; 1,3,5-tri-n-hexyl-2,4,6-triodobenzene; phosphonate/peptide conjugated iodobenzene derivatives. Iodine-containing nanoparticles may also be used as contrast agents. Nanoparticles may be administered in a wide variety of forms. Non-limiting examples of various forms of nanoparticle administration include lipsosomes, nanosuspensions, nanoemulsions, nanocapsules, micelles, among others. Liposomes may be spherical nanoparticles composed of a lipid bilayer and an aqueous inner core. Liposomes may comprise contrast agent molecules comprising iodine and antibodies (such as for example ICAM-1 monoclonal antibodies), and others. Contrast agent molecules maybe incorporated into a liposomal contrast agent in a variety of ways, including for example covalently binding to the lipid bilayer, or co-loading with contrast agent (such as iodinated poppyseed oil or ethiodol, for example). Nanosuspensions may be colloidal dispersions of pure drug particles stabilized by surfactants. An example of such, 6-ethoxy-6-oxohexyl 3,5-diacetamido-2,4,6-triiodobenzoate is a water-insoluble surfactant-stabilized crystalline substance designed as a macrophage-targeting CT contrast agent. Nanoemulsions may be stable nanostructures of one liquid material within an immiscible second liquid (e.g., oil in water). Non-limiting examples of nanoemulsions include: ethiodized oil, lipiodol, radioactively labeled lipiodol, 1,3-disubstituted polyiodinated triglycerides (ITG) in stealth or non-stealth formulations, poly(butadiene)-b-poly (ethylene oxide) (PBD-PEO) block-copolymer, in addition to others. Nanocapsules may be stable nanoparticles consisting of a crosslinked polymeric membrane enveloping a payload-material that is often insoluble/immiscible with the surrounding solvent. As a non-limiting example, a nanocapsule may be formed by cross-linking polymer around lipiodol oil nanodroplets. Polymeric nanoparticles may also be used as CT contrast agents. Polymeric molecules may comprise iodine or iodine-based contrast agent. Non-limiting examples of polymeric nanoparticles include 2-methacryloyloxyethyl-2,3,5-triiodobenzoate (MAOETIB)-glycidyl methacrylate (GMA) based nanoparticles and poly(vinyl alcohol) microparticles with iopamidol physically encapsulated in the structure. Nanomicelles may form from amphiphilic polymers in aqueous media. A non-limiting example of an amphiphilic polymer contrast agent capable of forming a nanomicelle is aliphatic 2,3,5-triiodobenzoyl substituted poly-L-lysine/MPEG copolymer.

Other CT contrast agents may include: barium sulfate, CA4+, iomeprol, any of the various lanthanide-based contrast agents noted above. Dendridimers may also be used as CT contrast agents. Dendridimers can be configured to exhibit high water solubility and good biocompatibility.

Non-limiting examples of other contrast agents may include: preparations containing barium sulfate; iodinated contrast agents such as diatrizoate meglumine, diatrizoate sodium, iothalamate meglumine, iothalamate sodium, ioxaglate meglumine sodium, iopamidol, ioversol, gadolinium-based agents such as Gadopentetate dimeglumine, nonionic Gd-HP-DOTA gadoteridol, nonionic gadoversetamide nonionic Gd-DTPA-GMA gadodiamide, gadobenate dimeglumine; paramagnetic agents such as nonionic ferrous-ferric oxide ferumoxsil and nonionic ferrous-ferric oxide ferumoxides.

Contrast agent may be delivered orally, intravenously or locally.

In any of the various aspects, a hypoxia-activated bio-reductive agent is administered to liver tissue of a subject. A hypoxia-activated bioreductive agent can include any chemical entity that, when administered to a patient, is an inactive prodrug that becomes activated only under hypoxic conditions. A hypoxia-activated bioreductive agent can become active in hypoxic environments. As a non-limiting example, hypoxia-activated bioreductive agent tirapazamine is activated by cytochrome P450 reductase by a one-electron reaction, thereby generating nitroxide radicals. In the absence of oxygen, nitroxide radicals induce single- and double-strand breaks in DNA to cause cell death. Other examples of hypoxia activated bioreductive agents include but are not limited to banoxantrone (AQ4N), porfiromycin, apaziquone (EO9), 1,2-bis(methylsulfonyl)-1-(2-chloroethyl)-2-[[1-(4-nitrophenyl)ethoxy]carbonyl]hydrazine (KS119), dinitrobenzamide mustard derivative (such as PR 104) and 4-[3-(2-nitro-1-imidazolyl)-propylamino]-7-chloroquinoline hydrochloride (NLCQ-1, NSC 709257).

In any of the various aspects, a therapeutically effective amount of a hypoxia-activated bioreductive agent is administered. The dosage of hypoxia-activated bioreductive agent that can be administered is in the range of from about 1 mg to about 200 mg (e.g. of tirapazamine), and preferably from about 5 to about 50 mg. The amount of hypoxia-activated bio-reductive agent may vary depending upon the subject and disease condition being treated, e.g., the weight and age of the subject, the severity of the disease condition, the manner of administration and the like, which can readily be determined by one of ordinary skill in the art.

Local application of a hypoxia-activated bioreductive agent is performed when the pressure of a hepatic artery is reduced. Reduction in arterial pressure may prolong the exposure to the hypoxia-activated bioreductive agent. In some embodiments, the hypoxia-activated bioreductive agent is applied after the arterial pressure is reduced. In other words, pressure of the hepatic artery may be reduced prior to applying the hypoxia-activated bioreductive agent. In other embodiments, pressure of the hepatic artery of the subject is reduced concurrent with applying the hypoxia-activated bioreductive agent. The hypoxia-activated bioreductive agent may be applied to the liver tissue after a period of time, which may be about 1 second or longer after arterial pressure reduction. After pressure reduction, application may take place after less than about 1 second. In some instances, the hypoxia-activated bioreductive agent may be applied after about 2-5, about 5 - 10, about 10 - 15, or about 15 or more seconds.

In some embodiments, in addition to locally applying a hypoxia-activated bioreductive agent to liver tissue under reduced pressure, liver tissue may be embolized. In further embodiments, embolizing may take place after applying a hypoxia-activated bioreductive agent.

Various techniques or devices may be used to administer an embolization agent. The anatomy of the blood supply of the region and the surrounding normal organs or tissues can determine whether the surrounding organs or tissues may experience significant damage due to lack of blood supply after embolization. An embolization agent may be administered using any of the techniques or devices for locally applying a hypoxia-activated bioreductive agent. An embolization agent may be administered using any of the techniques or devices for vessel occlusion. In some embodiments, administration of the embolizing agent may be performed by intra-arterial injection. Other methods for administering an embolization agent will be recognized by one having skill in the art. As a non-limiting example, embolization may be performed by placing a catheter from the femoral artery in the groin and advancing the tip of the catheter to the branch of the hepatic artery that supplies the tumor under fluoroscope x-ray guidance. Once the arterial branch supplying tumor is identified by injection of contrast material, embolizing agents such as Lipiodol or Gelfoam, may be injected.

Embolization may be permanent or temporary. In some embodiments, embolization is permanent, allowing the hypoxia activated bioreductive agent to be activated in the embolized hypoxic region for an extended period of time. In some embodiments, embolization is not permanent, and the duration of vascular occlusion can be controlled by the proper selection of embolizing agents, permitting the activated hypoxia-activated bioreductive agent to work for a period of time to execute the effect of tumor killing

Various embolization agents may be used to embolize the hepatic artery following local infusion of the hypoxia activated bioreductive agent. Embolization agents may be absorbable or non-absorbable. In some embodiments, Lipiodol or ethiodized oil may be used. Lipiodiol or ethiodized oil is radiopaque and may be used to visualize coverage of the embolization agent in liver tissue using, for example, cone beam computed tomography (CT). Non-limiting examples of other embolizing agents that may be used include but are not limited to gelfoam, gelatin, blood clots, microspherical beads, radioembolization microspheres, drug eluting beads, nanoparticles or any clinically proven mechanical agent that can achieve the purpose of vascular occlusion.

The dosage of embolization agent may vary widely. Appropriate embolization agent dosage will be recognized by one having skill in the art. An appropriate dosage may be a sufficient amount embolizing agent administered to achieve complete occlusion of an artery. Sufficient embolizing agent may be administered to achieve complete occlusion of the intended branch of the vessel under fluoroscope X-ray examination, cone beam CT, or other visualization technologies to ensure complete coverage in artery-supplied tissue, or the creation of a hypoxic region or condition in the embolized area. The dose of embolizing agent that may be administered may range from about 5-40, about 1-50, or about 1-100 mL. If embolization of the tissue is incomplete, it may be desirable to search for other arterial branches that supply the liver tissue or a particular lesion of interest and repeat the local infusion of hypoxia-activated bioreductive agent and embolization procedures. In some instances, the procedures may be repeated until embolization agent coverage is complete.

Methods of determining the most effective means and dosage of administration are well known to those of skill in the art and will vary with the composition used for therapy, the purpose of the therapy, the target cell or tissue being treated, and the subject being treated. Single or multiple administrations (e.g. about or more than about 1, 2, 3, 4, 5, 6, 7, 8, 9, 10, 11, 12, 13, 14, 15, 16, 17, 18, 19, 20, 25, 30, or more doses) can be carried out with the dose level and pattern being selected by the treating physician.

A hypoxia-activated bioreductive agent may be administered in any suitable amount, and in the order disclosed herein. In some embodiments, a hypoxia-activated bioreductive agent is administered to a subject within a range of about 1 mg-100mg per infusion, such as about, less than about, or more than about, 1mg, 2mg, 3mg, 4mg, 5mg, 6mg, 7mg, 8mg, 9mg, 10mg, 11mg, 12mg, 13mg, 14mg, 15mg, 16mg, 17mg, 18mg, 19mg, 20mg, 25mg, 30mg, 35mg, 40mg, 45mg, 50mg, 55mg, 60mg, 65mg, 70mg, 75mg, 80mg, 85mg, 90mg, 95 mg, or 100 mg per infusion.

The target dose may be administered in a single dose. Alternatively, the target dose may be administered in about or more than about 1, 2, 3, 4, 5, 6, 7, 8, 9, 10, 11, 12, 13, 14, 15, 16, 17, 18, 19, 20, 25, 30, or more doses.

In some instances, dosage levels below the lower limit of the aforesaid range may be more than adequate, while in other cases still larger doses may be employed without causing any harmful side effect.

In some embodiments, a hypoxia-activated bioreductive agent, and/or any additional therapeutic compound of the invention are administered in multiple doses or infusions. Dosing or infusions of hypoxia-activated bioreductive agent to liver tissue may be about once, twice, three times, four times, five times, six times, or more than six times. Dosing may be about once a month, once every two weeks, once a week, or along with each embolization. In some embodiments, cycles of administering a hypoxia-activated bioreductive agent followed by one or more administrations of an embolization agent are repeated until liver tissue undergoes necrosis. In some cases, repetition of a dosing cycle comprising administration of a hypoxia-activated bioreductive agent followed by one or more embolizations is continued as long as necessary.

Administration of the combination treatments of the invention may continue as long as necessary. In some embodiments, a hypoxia-activated bioreductive agent and/or an embolization agent are administered more than 1, 2, 3, 4, 5, 6, 7, or 10 times, wherein an administration of the embolization agent is subsequent to an administration of the hypoxia-activated bioreductive agent. In some embodiments, a hypoxia-activated bioreductive agent and/or an embolization agent of the invention is administered for less than 10, 7, 6, 5, 4, 3, 2, or 1 times, wherein an administration of the embolization agent is subsequent to an administration of the hypoxia-activated bioreductive agent. In some embodiments, a hypoxia-activated bioreductive agent and/or an embolization agent of the invention is administered chronically on an ongoing basis, e.g., for the treatment of chronic effects, wherein an administration of the embolization agent is subsequent to an administration of the hypoxia-activated bioreductive agent.

When a combination treatment of the invention is administered as a composition that comprises one or more compounds, and one compound has a shorter half-life than another compound, the unit dose forms may be adjusted accordingly.

### Example

According to the following example, local infusion of a hypoxia-activated bioreductive agent when hepatic arterial pressure is reduced and subsequent embolization will cause liver tumor necrosis. A 4-French angiocatheter will cannulate the major branch of the hepatic artery that supplies a targeted liver cancer lesion. A balloon microcatheter (3-French or smaller) will then be inserted through the 4-French angiocatheter already in place. The small microcatheter will then be cannulated into the arterial branch that supplies the targeted lesion. An arteriogram will be performed to ensure that the position of the tip of microcatheter in the right position for subsequent injection of hypoxia-activating agent followed by Lipiodol. Once it is verified that the balloon catheter is appropriately positioned, the balloon will be inflated to stop or slow down blood flow thereby reducing the pressure of the artery beyond the tip of balloon. Hypoxia-activated bioreductive agent tirapazamine will be injected in a calculated volume appropriate for the target tumor lesion through the microcatheter and will be applied to the tumor. The calculated volume of tirapazamine will be an amount sufficient for complete coverage of the targeted tumor lesion. Applied tirapazamine will diffuse to the tumor. The decreased blood pressure and flow rate in the artery will increase the duration of tissue exposure to the applied hypoxia-activated bioreductive agent which will thereby enhance delivery of the agent to the tissue.

Following diffusion, the tissue will then be embolized. Embolization will provide prolonged vessel occlusion such that liver tissue may become hypoxic or ischemic thereby activating hypoxia-activated bioreductive agent. Embolizing a hepatic arterial branch in the liver tissue when blood pressure is reduced will decrease potential for backflow of the embolization agent to other arterial branches. Backflow can result in complications such as undesirable vessels occlusion, ischemia, and necrosis. Additionally, embolization under reduced arterial pressure will result in more complete embolization thereby enhancing hypoxia-activated bioreductive agent activity and increasing efficacy. Thus, embolization will provide a synergistic effect when combined with or following local infusion of hypoxia-activated bioreductive agent under reduced pressure. Namely, reduced arterial pressure prolongs the duration of hypoxia-activated bioreductive agent exposure and embolization enhances the efficacy of the hypoxia-activated bioreductive agent by further inducing hypoxic conditions.

Embolization is to be visualized using real-time x-ray monitoring. Embolization agent Lipiodol (ethiodized oil) is to be injected through the balloon microcatheter. A sufficient amount of embolization agent will be administered to block blood flow and induce tumor hypoxia. The balloon of the balloon microcatheter will be deflated after embolization injection is complete. Visualization using cone beam CT should show embolization agent (i.e. Lipiodol) coverage in the tumor lesion. Alternately, where cone beam CT is not available for visualization, arteriogram can be used to visualize embolization agent coverage. If embolization agent coverage is complete, the catheters will be withdrawn. If embolization agent coverage in the tumor lesion is incomplete, an interventional radiologist will search for other arterial branches that supply the tumor and repeat the process of positioning the balloon microcatheter, inflating the balloon, locally infusing a tirapazamine, allowing for diffusion, embolizing the tissue, and checking for coverage.

While preferred embodiments of the present invention have been shown and described herein, it will be obvious to those skilled in the art that such embodiments are provided by way of example only. It should be understood that various alternatives to the embodiments of the invention described herein may be employed in practicing the invention, provided that they are covered by the claims. It is intended that the following claims define the scope of the invention and that methods and structures within the scope of these claims be covered thereby Any references to methods of treatment in the foregoing paragraphs of this description are to be interpreted as references to the compounds, pharmaceutical composition or medicaments of the present invention for use in a method of treatment of a subject by therapy.

## Claims

1. A hypoxia-activated bioreductive agent for use in a method of administering said agent to a liver tissue of a subject in need thereof to treat liver cancer, the method comprising:
applying locally to said liver tissue a therapeutically effective amount of said agent, wherein said applying locally is performed when pressure of a hepatic artery of said subject is reduced, wherein said pressure is reduced by introducing a vessel occlusion device comprising a balloon into said hepatic artery, thereby treating said liver cancer.

2. The agent for use of claim 1, wherein said applying locally is performed in said hepatic artery.

3. The agent for use of claim 1 or 2, wherein said pressure of said hepatic artery is reduced proximal to said liver tissue.

4. The agent for use of any one of claims 1-3, wherein said pressure of said hepatic artery is reduced by at least about 20% or hepatic artery blood flow volume is reduced by at least about 20%.

5. The agent for use of any one of claims 1-4, wherein said pressure of said hepatic artery is reduced by inflating said balloon.

6. The agent for use of any one of claims 1-5, wherein the local application is performed with the aid of a balloon catheter.

7. The agent for use of claim 6, wherein said balloon catheter comprises an external diameter of 1 mm or less.

8. The agent for use of claim 6 or 7, wherein said hypoxia-activated bioreductive agent is (i) applied from a distal tip of said balloon catheter, and (ii) diffused to said liver tissue.

9. The agent for use of any one of claims 1-8, wherein said pressure of said hepatic artery of said subject is reduced prior to or concurrent with applying said hypoxia-activated bioreductive agent.

10. The agent for use of any one of claims 1-9, further comprising embolizing said liver tissue.

11. The agent for use of claim 10, wherein the step of embolizing takes place after the step of applying of said hypoxia-activated bioreductive agent.

12. The agent for use of claim 10 or 11, wherein the step of embolizing said liver tissue is performed with an absorbable or non-absorbable embolization agent selected from the group consisting of ethiodized oil, absorbable gelatin, and non-absorbable microspherical beads.

13. The agent for use of any one of claims 1-12, wherein said hypoxia-activated bioreductive agent is tirapazamine.

14. The agent for use of claim 13, wherein about 1 mg to about 200 mg of said tirapazamine are applied to said subject.

## Patentansprüche

1. Hypoxie-aktiviertes bioreduktives Mittel zur Verwendung in einem Verfahren zur Verabreichung des Mittels an ein Lebergewebe eines bedürftigen Individuums zur Behandlung von Leberkarzinomen, wobei das Verfahren Folgendes umfasst:
das lokale Anwenden einer therapeutisch wirksamen Menge des Mittels auf das Lebergewebe, wobei das lokale Anwenden bei verringertem Druck einer Leberarterie des Individuums durchgeführt wird, wobei der Druck durch Einführen einer Gefäßverschlussvorrichtung, die einen Ballon umfasst, in die Leberarterie verringert wird, wodurch die Leberkarzinome behandelt werden.

2. Mittel zur Verwendung nach Anspruch 1, wobei das lokale Anwenden in der Leberarterie durchgeführt wird.

3. Mittel zur Verwendung nach Anspruch 1 oder 2, wobei der Druck der Leberarterie in der Nähe des Lebergewebes verringert wird.

4. Mittel zur Verwendung nach einem der Ansprüche 1 bis 3, wobei der Druck der Leberarterie um zumindest 20 % verringert wird oder das Blutstromvolumen der Leberarterie um zumindest 20 % verringert wird.

5. Mittel zur Verwendung nach einem der Ansprüche 1 bis 4, wobei der Druck der Leberarterie durch Aufblasen des Ballons verringert wird.

6. Mittel zur Verwendung nach einem der Ansprüche 1 bis 5, wobei das lokale Anwenden mit Hilfe eines Ballonkatheters durchgeführt wird.

7. Mittel zur Verwendung nach Anspruch 6, wobei der Ballonkatheter einen Außendurchmesser von 1 mm oder weniger aufweist.

8. Mittel zur Verwendung nach Anspruch 6 oder 7, wobei das Hypoxie-aktivierte bioreduktive Mittel (i) aus einer distalen Spitze des Ballonkatheters angewendet und (ii) in das Lebergewebe verteilt wird.

9. Mittel zur Verwendung nach einem der Ansprüche 1 bis 8, wobei der Druck der Leberarterie des Individuums vor oder während des Anwendens des Hypoxie-aktivierten bioreduktiven Mittels verringert wird.

10. Mittel zur Verwendung nach einem der Ansprüche 1 bis 9, das außerdem das Embolisieren des Lebergewebes umfasst.

11. Mittel zur Verwendung nach Anspruch 10, wobei der Schritt des Embolisierens nach dem Schritt des Anwendens des Hypoxie-aktivierten bioreduktiven Mittels erfolgt.

12. Mittel zur Verwendung nach Anspruch 10 oder 11, wobei der Schritt des Embolisierens des Lebergewebes mit einem absorbierbaren oder nicht absorbierbaren Embolisierungsmittel durchgeführt wird, das aus der aus Ethiodatöl, absorbierbarer Gelatine und nicht absorbierbaren Mikrokügelchen bestehenden Gruppe ausgewählt ist.

13. Mittel zur Verwendung nach einem der Ansprüche 1 bis 12, wobei das Hypoxie-aktivierte bioreduktive Mittel Tirapazamin ist.

14. Mittel zur Verwendung nach Anspruch 13, wobei etwa 1 mg bis etwa 200 mg Tirapazamin auf das Individuum angewendet werden.

## Revendications

1. Agent bioréducteur activé par hypoxie à utiliser dans un procédé d'administration dudit agent à un tissu hépatique d'un sujet qui en a besoin pour traiter un cancer du foie, le procédé comprenant les étapes consistant à :
appliquer localement audit tissu hépatique une quantité thérapeutiquement efficace dudit agent, dans lequel ladite application locale est effectuée lorsque la pression de l'artère hépatique dudit sujet est réduite, dans lequel ladite pression est réduite en introduisant un dispositif d'occlusion de vaisseau comprenant un ballonnet dans ladite artère hépatique, en traitant ainsi ledit cancer du foie.

2. Agent à utiliser selon la revendication 1, dans lequel ladite application locale est effectuée dans ladite artère hépatique.

3. Agent à utiliser selon la revendication 1 ou 2, dans lequel ladite pression de ladite artère hépatique est réduite à proximité dudit tissu hépatique.

4. Agent à utiliser selon l'une quelconque des revendications 1 à 3, dans lequel ladite pression de ladite artère hépatique est réduite d'au moins environ 20 % ou le volume d'écoulement sanguin de l'artère hépatique est réduit d'au moins environ 20 %.

5. Agent à utiliser selon l'une quelconque des revendications 1 à 4, dans lequel ladite pression de ladite artère hépatique est réduite en gonflant ledit ballonnet.

6. Agent à utiliser selon l'une quelconque des revendications 1 à 5, dans lequel l'application locale est effectuée à l'aide d'un cathéter à ballonnet.

7. Agent à utiliser selon la revendication 6, dans lequel ledit cathéter à ballonnet présente un diamètre externe de 1 mm ou moins.

8. Agent à utiliser selon la revendication 6 ou 7, dans lequel ledit agent bioréducteur activé par hypoxie est (i) appliqué à partir d'une pointe distale dudit cathéter à ballonnet, et (ii) diffusé dans ledit tissu hépatique.

9. Agent à utiliser selon l'une quelconque des revendications 1 à 8, dans lequel ladite pression de ladite artère hépatique dudit sujet est réduite avant ou pendant l'application dudit agent bioréducteur activé par hypoxie.

10. Agent à utiliser selon l'une quelconque des revendications 1 à 9, comprenant en outre une embolisation dudit tissu hépatique.

11. Agent à utiliser selon la revendication 10, dans lequel l'étape d'embolisation a lieu après l'étape d'application dudit agent bioréducteur activé par hypoxie.

12. Agent à utiliser selon la revendication 10 ou 11, dans lequel l'étape d'embolisation dudit tissu hépatique est effectuée avec un agent d'embolisation absorbable ou non absorbable choisi dans le groupe comprenant de l'huile éthiodée, de la gélatine absorbable et des billes microsphériques non absorbables.

13. Agent à utiliser selon l'une quelconque des revendications 1 à 12, dans lequel ledit agent bioréducteur activé par hypoxie est la tirapazamine.

14. Agent à utiliser selon la revendication 13, dans lequel environ 1 mg à environ 200 mg de ladite tirapazamine sont appliqués audit sujet.
